# EUROPEAN PATENT APPLICATION

(11) **EP 1 742 054 A1**
(43) Date of publication of application: **10.01.2007**
(21) Application number: 05736647.8
(22) Date of filing: 21.04.2005
(51) Int. Cl.: G01N 33/53, G01N 21/27, G01N 21/35, G01N 21/78, G01N 33/566

(54) **BIOCHIP PRODUCING METHOD, BIOCHIP, BIOCHIP ANALYZING DEVICE, BIOCHIP ANALYZING METHOD**

(30) Priority: 28.04.2004 JP 2004133877
(71) Applicant: Japan Science and Technology Agency, Kawaguchi-shi, Saitama 332-0012 (JP)
(72) Inventor: URISU, Tsuneo, Okazaki-shi, Aichi 444-0005 (JP); SUZUI, Kouichi, 2-54 Tatsumigaoka-koumuinsyukusya, Okazaki-shi, Aichi 444-0874 (JP)
(74) Representative: Kuhnen & Wacker
(86) International application number: PCT/JP2005/008312
(87) International publication number: WO 2005/106472

(57) **Abstract**

A biochip to be used for infrared reflection absorption spectroscopy analysis, which is obtainable by forming a metallic layer and an active layer made from a nonmetallic material and has a film thickness less than an infrared wavelength and remarkably flat surface on a solid base and immobilizing a probe on the active layer. A biochip analysis apparatus including the biochip, a liquid passing unit for supplying a sample liquid containing a test substance to the biochip, and an optical system entering infrared light to the biochip and detecting a reflected infrared light. A biochip analysis method using the apparatus.

As described above, the novel biochip that is useful for infrared reflection absorption spectroscopy analysis, the biochip analysis apparatus incorporating the biochip, and the biochip analysis method are provided.

## Description

### BACKGROUND OF THE INVENTION

### Field of the Invention

This invention relates to a biochip for detecting presence or absence of a test substance in a sample. More specifically, this invention relates to a novel biochip production method, an excellent biochip produced by the method, a biochip analysis apparatus realized by incorporating the biochip, and a biochip analysis method using the biochip analysis apparatus. Representative examples of the "biochip" of this invention include a protein chip, a nucleic acid chip (DNA chip), a cell chip, and the like.

### Description of the Related Art

As an analysis method of a protein chip, the Surface Plasmon Resonance method (SPR method), the Enzyme-Linked ImmunoSorbent Assay method (ELISA method), and the like have heretofore been known. The protein chip is a chip used for confirming the presence or absence of a test substance reacting with a specific protein. The SPR method is a method for considering a change in physical property value caused on a protein chip surface by a reaction between a protein on the protein chip surface and a test substance. The ELISA method is a method for detecting emission of fluorescence or radioactive ray resulting from a reaction with a test substance. One example of the SPR method is disclosed in Literature 1 indicated below.

### [Literature 1]

"Real-Time Analytic Experiment Method of Biomaterial Interaction"
Edited by Kazuhiro Nagata and Hiroshi Handa; Springer-Verlag Tokyo

However, it takes a lot of time and effort to carry out all the steps of the above-described conventional detection methods using a color reaction and a chemical reaction in the protein chip, and it is difficult to conduct the method using a large number of protein chips.

Further, though it is possible to judge whether or not the test substance is adsorbed by a probe protein with the SPR method and the ELISA method, it is impossible to examine a molecular structure and the like of the protein with the methods. Therefore, when the adsorption occurs, a reaction which is not the original detection object is detected. Therefore, a problem of insufficient accuracy of detection and measurement has been raised. A lot of time and effort as well as a large system are required also for carrying out all the steps with the method, thereby entailing a high cost.

Accordingly, a protein chip and a biochip capable of enabling analysis using optical technologies such as infrared spectroscopy analysis are being noted. For example, Literature 2 indicated below discloses one example of antibody chip using Fourier transform reflection infrared spectroscopy. In this antibody chip, a self assembled monolayer (SAM) is formed on a surface treated gold plated glass slide, and then a specific antibody is immobilized on the SAM.

### [Literature 2]

Chemical Society of Japan Lecture Essays, Vol. 84, No. 1 (2004), A-7, "Development of Next Generation Chip Aimed for Simplified Diagnosis" by Shigeori Takenaka

Literature 3 indicated below also discloses a similar molecular array. In this molecular array, various peptides immobilized on a substrate are interacted with a protein in a biological sample. It is mentioned in Literature 3 that this interaction can be detected by using a microscopic Fourier transform infrared spectroscopy apparatus and by using infrared absorption of a carbonyl group as an index. Also, it is mentioned that it is possible to immobilize a protein, nucleic acid, or the like on a gold plated glass substrate by way of bonding between a thiol group and gold.

### [Literature 3]

JP-A-2004-45390
"Analysis Method of Test Substance by Molecular Array using Spectroscopy"

The technologies disclosed in Literature 2 and Literature 3 leverage easy deposition of the self assembled monolayer of the thiol-based molecule on the surface of gold. However, in recent years, it is known to persons skilled in the art that the biochip of the above type has serious drawbacks that can degrade its function and reliability.

The first drawback is inactivation of the functional protein immobilized as a probe on the gold surface and deactivation (or death) of cells. That is, by directly contacting the active functional protein or living cells to gold, the protein is inactivated or the cellular activity is deactivated. In such cases, the protein or the cells do not function as the probe.

The second drawback is more essential, and it is difficult to overcome this drawback. That is, according to experiences of the inventors of this patent application, it is difficult to keep a flatness of the gold surface in terms of difference of elevation to several tens of nanometers or less by employing the conventional chip production method. However, as a diameter of serum albumin is about 5 nm, for example, typical functional proteins have the size of 10 nm or less. Ordinary probe nucleic acids have a smaller size. As a result, the probe is immobilized on the base having the surface irregularity larger than its size. Accordingly, orientation and posture when immobilizing the probe-will be considerably random. Consequently, bonding efficiency of the probe to a test substance is low, and the bonding efficiency is fluctuated among individual chip products, thereby resulting in a low reliability as a biochip.

The above drawbacks have been noted among persons skilled in the art particularly recently. For example, in the second paragraph of the item "1.2.1.7.2" on page 27 of Literature 4 indicated below, it is mentioned "... in the case of using proteins containing an enzyme as a measurement obj ect, since the proteins have the size of 10 nm, it is difficult to conduct the measurement on a plate-like kit such as a chip even if the proteins can be captured. More specifically, irregularity of more than 100 nm is formed on a surface of an ordinary slide glass or a plastic, and the proteins of the measurement object are entrapped in the depressions, thereby causing fluctuation and sensitivity degradation in detection".

### [Literature 4]

Issued by Mechanical Social Systems Foundation on March, 2003; Consigned to: Japan Bioindustry Association "Report on Technologies and Marketability for Nanobiomachine Production"

### SUMMARY OF THE INVENTION

An object of this invention is to provide a biochip and its production method capable of solving the first and the second drawbacks. Another object of this invention is to provide various biochips that are capable of solving the first and the second drawbacks; achieving general versatility; downsizing an apparatus and reducing the number of steps; and ensuring accuracy of measurement as well as to provide an analysis unit for such biochips.

According to the first aspect of this invention, there is provided a biochip production method comprising the steps of:
(1) forming a metallic layer on a solid base;
(2) depositing a non-metallic material on the metallic layer by vapor deposition, sputtering, or chemical vapor deposition (CVD) and then forming, by performing annealing, an active layer made from a nonmetallic material and having a film thickness lower than an infrared wavelength and a surface flatness in terms of difference of elevation of 5 nm or less; and
(3) immobilizing a probe on the surface of the active layer.

According to the first aspect of this invention, a protein or a living cell which is the probe does not directly contact a metal such as gold since the active layer of the nonmetallic material is formed on the metallic layer. Therefore, there is little or no fear of causing inactivation of the protein as the probe and deactivation of activity of the cell as the probe.

Also, by performing the annealing after depositing the nonmetallic material by an appropriate means on the metallic layer in forming the active layer, the metallic layer and the active layer above the metallic layer are formed remarkably flat. The flatness in terms of difference of elevation is ordinarily 5 nm or less. Particularly, in the case of depositing an amorphous nonmetallic material on the metallic layer, it is possible to achieve a difference of elevation of 1 nm. The probe to be immobilized on the surface of such active layer is immobilized with a fixed orientation and posture with being little influenced by irregularity on the immobilization surface. Therefore, the probe is bound to a test substance at high efficiency, and binding efficiency of each of the biochips becomes stable, thereby enhancing the reliability.

A material used for the metallic layer is not particularly limited, and cobalt, cobalt silicide, platinum, gold, silver, and the like and an alloy thereof may be used for the metallic layer. Particularly, cobalt is preferable. In the case of using the metallic material other than cobalt, such as gold or silver, though no adverse effect is caused on the original function of the metallic layer, the metallic layer can be easily stripped off from the nonmetallic material layer in the annealing in some cases. In such case, a second metallic layer such as Cr may be inserted between the metallic layer and the nonmetallic material layer for the purpose of overcoming the problem of stripping off easily.

According to the second aspect of this invention, the annealing according to the first aspect of this invention is performed under an inert gas atmosphere at a temperature of 300°C to 800°C.

That is, as conditions of the annealing, the conditions of under the inert gas atmosphere and at 300°C to 800°C may particularly be preferred. By performing the annealing under an aerobic condition, there is fear that greater irregularity can be easily formed due to the surface of the active layer (immobilization surface). When the annealing is performed under a condition of lower than 300°C, there is fear that the metallic layer and the active layer (nonmetallicmaterial layer) become fragile to be easily stripped off in a chemical treatment performed subsequently. In turn, when the annealing is performed under a condition of higher than 800°C, there is fear that the metallic layer and the active layer are destroyed.

According to a third aspect of this invention, a semiconductor material or an electrical insulating material is used as the nonmetallic material according to the first or the second aspect of this invention.

As the nonmetallic material for forming the active layer, an amorphous nonmetallic material may be more preferable. Though the type of the nonmetallic material is not limited, it is preferable to use the semiconductor material or the electrical insulating material in view of prevention of deactivation of the probe, easiness of a chemical treatment for immobilizing the probe, and resistance to chemical drugs that the active layer is required to achieve.

According to a fourth aspect of this invention, there is provided a biochip comprising:
a solid base; a metallic layer provided on the solid base; an active layer provided on the metallic layer and made from a nonmetallic material; and a probe immobilized on a surface of the active layer, wherein
the active layer has a film thickness less than an infrared wavelength and a surface flatness in terms of difference of elevation of 5 nm or less.

The biochip according to the fourth aspect of this invention has the active layer made from the nonmetallic material on the metallic layer as described in conjunction with the first aspect of this invention. Therefore, it is possible to effectively prevent inactivation of a protein as the probe and deactivation of activity of a cell as the probe. Also, since the surface of the active layer is remarkably flat, binding efficiency of the immobilized probe to a test substance is high, and the biochip is highly reliable since the binding efficiencies of a plurality of such biochips are constant.

Also, since the biochip according to the fourth aspect of this invention is a chip for conducting infrared reflection absorption spectroscopy analysis, it is possible to identify the test substance from comparison between infrared reflection absorption spectrums before and after the binding of the probe to the test substance. Therefore, the biochip is more convenient since the number of measurement steps is smaller than that of conventional biochips utilizing color reactions or chemical reactions. Further, since it is possible to identify the test substance, accuracy of detection and measurement is excellent.

Further, it is possible to form the active layer having the film thickness less than the infrared wavelength, more preferably having a sufficiently small film thickness, and to provide the metallic layer under the active layer as a metallic layer of an embedded type. In this case, the chip imparts properties of the metallic layer to the infrared light to contribute to effective infrared reflection absorption spectroscopy analysis.

It is possible to form this biochip at a considerably low cost as compared to a prism. Therefore, in the case of incorporating an expensive prism into an optical system of an infrared reflection absorption spectroscopy_ analysis apparatus using the biochip, it is sufficient to use one prism, and it is possible to conduct many test substance analyses by using a large number of the low cost biochips.

According to a fifth aspect of this invention, the probe according to the fourth aspect of this invention is a protein, a polynucleotide, a sugar chain, or a cell.

Examples of the biochip of this invention may preferably be a protein chip, a nucleic acid chip (DNA chip), a sugar chain chip, and a cell chip as defined in the fifth aspect of this invention. Though the type of the protein is not limited in the fifth aspect of this invention, the protein includes a protein which is an antigen or an antibody, a protein which is a receptor, and the like, and, in a broad sense, the protein includes a protein capable of specifically binding to a specific ligand. As used herein, the ligand is a concept including various organic or inorganic chemical substances, proteins, and various cells such as a cancer cell. The polynucleotide includes DNA and RNA which are not limited to single chain type. As the cell, a living cell is particularly useful.

According to a sixth aspect of this invention, the active layer according to the fourth or fifth aspect of this invention is made from a semiconductor material or an electrical insulating material.

More specifically, though the material used for the active layer is not particularly limited insofar as the material is the nonmetallic material, the semiconductor material or the electrical insulating material are preferred from the reasons described in conjunction with the third aspect of this invention.

According to a seventh aspect of this invention, the active layer according to any one of the fourth to sixth aspects of this invention has a film thickness of 200 nm or less in the case of using p-polarization infrared light and has a film thickness of 600 nm or more in the case of using an s-polarization infrared light.

The active layer has the film thickness less than the infrared wavelength. More specifically, in view of the fact that detection sensitivity is enhanced when a phase shift between incident light and reflected light on the immobilized protein surface is 0 or a value close to 2π, the film thickness of 200 nm or less is preferred in the protein chip compatible with the use of the p-polarization infrared light. Also, from the same reason, the film thickness of 600 nmormore is preferred in the protein chip compatible with the use of the s-polarization infrared light.

From a different point of view, since it is easy to increase the thickness of the active layer of the biochip of this invention to more than 600 nm, it is possible to perform a spectrum measurement with s-polarization as described later in Example 3 and to identify the protein or the like with higher accuracy.

According to an eighth aspect of this invention, a modification layer having a reaction group is formed on a surface portion of the active layer according to any one of the fourth to seventh aspects of this invention, and the probe is immobilized by a chemical binding with the reaction group.

The method of immobilizing the probe protein to the surface of the active layer is not particularly limited. One of preferred methods is the chemical modification. That is, the modification layer having the reaction group is formed on the surface of the active layer, and the probe protein is immobilized by the chemical binding with the reaction group.

According to a ninth aspect of this invention, in the case where the probe according to any one of the fourth to eighth aspects of this invention is the protein, a lipid double membrane is formed on the surface of the active layer, and the probe is immobilized in a state where the probe is embedded in the lipid double membrane.

As a preferred method of immobilizing the probe protein on the surface of the active layer, a method of forming the lipid double membrane on the surface of the active layer and then immobilizing the probe protein in the state where the probe protein is embedded in the lipid double membrane may be performed. When performing this method, a hydrophobic region of the probe protein is embedded in the lipid double membrane with the hydrophobic region penetrating through the lipid membrane, so that it is possible to control orientation of the immobilized probe protein. This method is particularly suitable for immobilizing an ion channel that can easily be inactivated due to its complicated structure and a lipid membrane penetration type protein such as a receptor protein.

The method of using such lipid double membrane has heretofore been proposed as an excellent method of immobilizing a probe protein with a constant orientation or posture. However, in the conventional protein chips, since the surface of the base layer of the lipid double membrane has great irregularity as described in the foregoing, the lipid double membrane inevitably has the great irregularity. In the lipid double membrane having the great irregularity, it is difficult to immobilize the probe protein with constant orientation or posture.

According to a tenth aspect of this invention, a spacer layer formed by hollowing out a space for liquid reservoir formation is further provided on the active layer according to any one of the fourth to ninth aspects of this invention.

In the case of using the biochip having further the spacer layer formed by hollowing out the space for the liquid reservoir formation on the active layer, it is possible to introduce a test substance (protein, for example) into the liquid reservoir in a state of a sample liquid which is hardly degenerated. Therefore, it is possible to bring the test substance as the sample liquid into contact with the probe for the infrared reflection absorption spectroscopy analysis.

According to an eleventh aspect of this invention, the spacer layer according to the tenth aspect of this invention has a uniform thickness in the range of 0.2 to 100 µm.

The thickness of the spacer layer (depth of the liquid reservoir) may preferably be in the range of 0.2 to 100 µm, more preferably in the range of 0. 2 to 10 µm. When the thickness of the spacer layer exceeds 100 µm, infrared light is absorbed by water in the liquid reservoir. Accordingly, there is fear that a noise near 1, 700 cm⁻¹ which is crucial to the proteins is increased to make the measurement difficult. When the thickness is less than 0.2 µm, there is fear that flow of the sample liquid in the liquid reservoir is prevented.

According to a twelfth aspect of this invention, there is provided a biochip analysis apparatus comprising:
the biochip defined in any one of the fourth to eleventh aspects of this invention, a liquid passing unit for supplying a sample liquid containing a test substance to the biochip; and an optical system entering infrared light to the biochip and detecting reflected infrared light.

Since the biochip analysis apparatus according to the twelfth aspect of this invention comprises the biochip, the liquid passing unit, and the optical system, troublesome and expensive reaction/analysis systems that are used in the case of employing color reaction and chemical reaction are not required, and it is possible to promptly obtain results of the measurement and analysis.

According to a thirteenth aspect of this invention, the optical system according to the twelfth aspect of this invention further comprises a prism provided on the biochip.

In the biochip analysis apparatus according to the twelfth aspect of this invention, it is preferable to comprise the prism provided on the biochip as described later in this specification.

According to a fourteenth aspect of this invention, the biochip according to the twelfth or thirteenth aspect of this invention is disposed in a space capable of realizing a substantially vacuum state.

By disposing the biochip in the substantially vacuum state, it is possible to measure the infrared absorption spectrum without being influenced by water and carbon dioxide gas in the atmosphere. Accordingly, it is possible to achieve highly sensitive measurement. Also, it is possible to perform spectroscopy in a low frequency region of several hundreds cm⁻¹ such as in a terahertz region in which the prism or the like cannot be used due to increased absorption by substances.

According to a fifteenth aspect of this invention, the liquid passing unit according to any one of the twelfth to the fourteenth aspects of this invention is formed of the liquid reservoir in the biochip provided with the spacer layer and a liquid passing structure provided with a liquid passage for supplying and discharging the sample liquid to and from the liquid reservoir.

Though the structure of the liquid passing unit provided in the biochip analysis apparatus is not limited, it is preferable that the liquid passing unit is formed of the liquid reservoir formed by the spacer layer of the biochip and the liquid passing structure provided with the liquid passage for supplying and discharging the sample liquid to and from the liquid reservoir as in the fifteenth aspect of this invention.

According to a sixteenth aspect of this invention, there is provided a biochip analysis method using the biochip analysis apparatus according to any one of the twelfth to fifteenth aspects of this invention, comprising the steps of:
(1) measuring, as a background of an infrared reflection absorption spectrum, reflected infrared light of infrared light entered from the optical system of the biochip analysis apparatus to a biochip which has not contacted a test substance;
(2) supplying a sample liquid to the biochip to cause the test substance in the sample liquid to be adsorbed when contacting a probe and then measuring reflected infrared light of infrared light entering the biochip as a signal of an infrared reflection absorption spectrum; and
(3) obtaining a spectrum which is a ratio between the signal and the background and then identifying the test substance by using the spectrum as an infrared reflection absorption spectrum of the test substance.

The biochip analysis method according to the sixteenth aspect of this invention identifies the test substance from the spectrum which is the ratio between (1) the infrared reflection absorption spectrum as the background and (2) the infrared reflection absorption spectrum as the signal. Therefore, the analysis is easy and rapid. Also, since it is possible to exclude an adsorption reaction which is not the detection object, the analysis is accurate.

According to a seventeenth aspect of this invention, the entering of the infrared light and the collection of the reflected infrared light of (1) and (2) according to the sixteenth aspect of this invention are performed via the prism.

In the above-described biochip analysis method, it is preferable to perform the entering of the infrared light to the measurement object and the collection of the reflected infrared light via the prism.

According to an eighteenth aspect of this invention, an incident angle in entering the infrared light to the biochip according to the sixteenth or the seventeenth aspect of this invention is set to 60° or more of grazing incidence.

It is preferable to set the incident angle of the infrared light to the biochip to 60° or more of grazing incidence. When the incident angle is excessively large in using the prism, the infrared light is reflected at the bottom of the prism. As a result, there is fear that the infrared light reaching the biochip is weakened to reduce the sensitivity.

According to a nineteenth aspect of this invention, the sample liquid of (2) according to any one of the sixteenth to eighteenth aspects of this invention is supplied to the liquid reservoir in the biochip provided with the spacer layer, and the process in which the infrared light entered to the liquid reservoir and reflected by the biochip is further guided to the liquid reservoir so as to cause the infrared light to be reflected by the biochip is repeated at least once.

By repeating the infrared light reflection as in the nineteenth aspect of this invention, the absorption spectrum is intensified to enhance the measurement sensitivity.

According to a twentieth aspect of this invention, the unit for guiding the reflected infrared light according to the nineteenth aspect of this invention further to the liquid reservoir is the prism provided on the biochip.

It is possible to perform the repeat of the infrared light reflection as in the nineteenth aspect of this invention by the prism provided on the biochip.

The above and other advantages of the invention will become more apparent in the following description and the accompanying drawings.

### BRIEF DESCRIPTION OF THE DRAWINGS

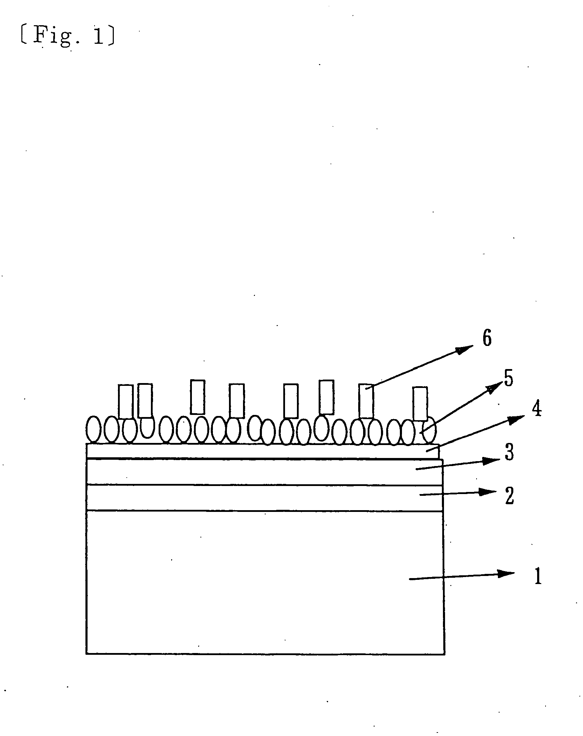

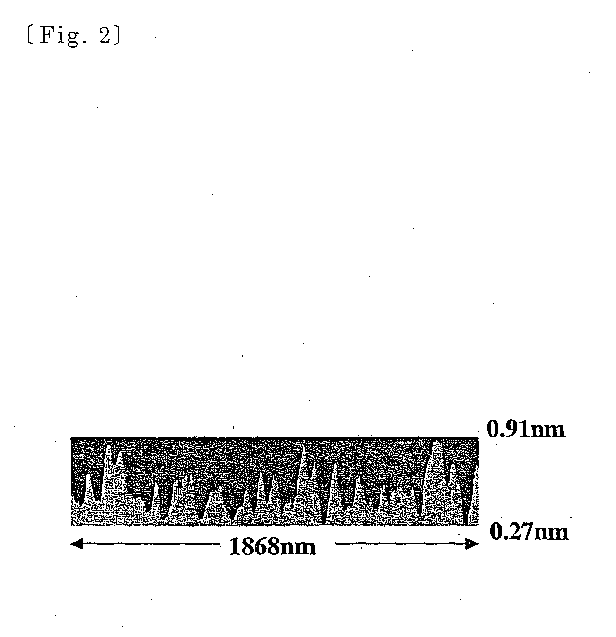

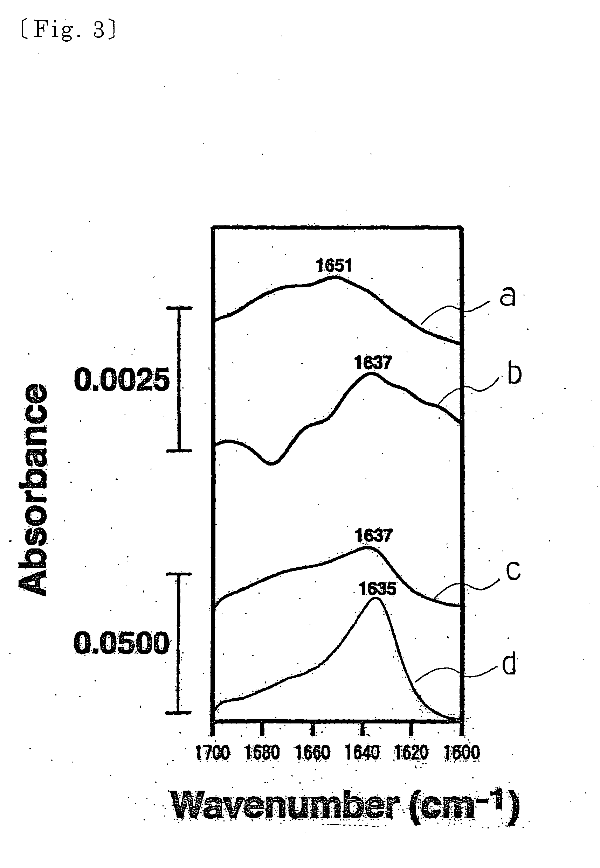

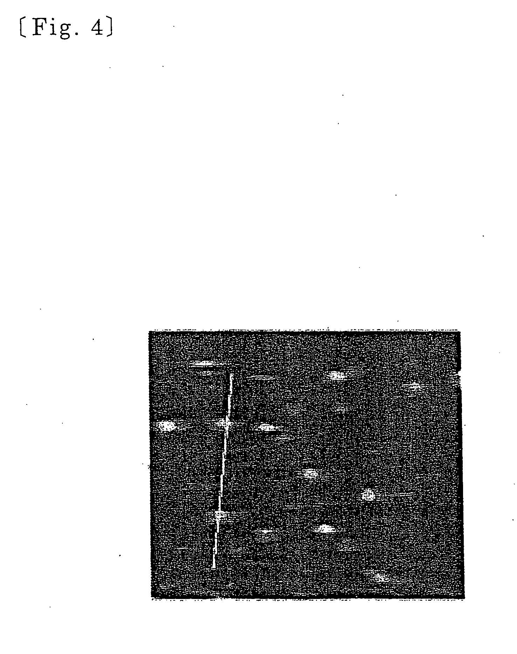

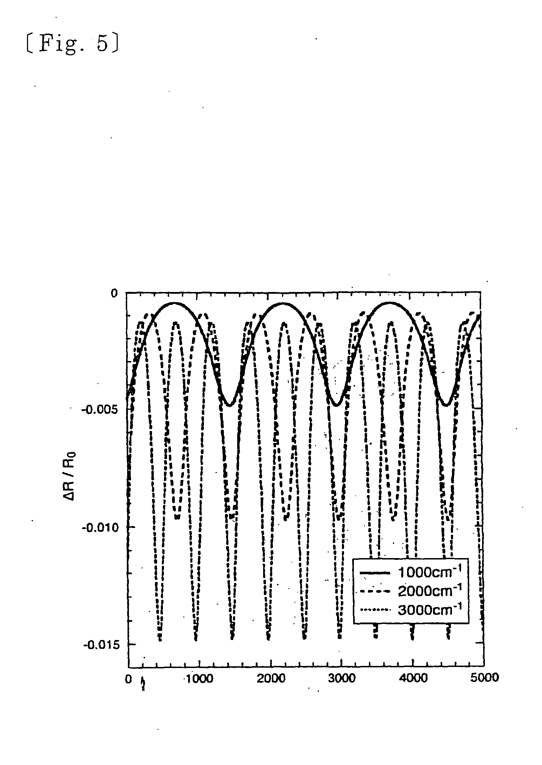

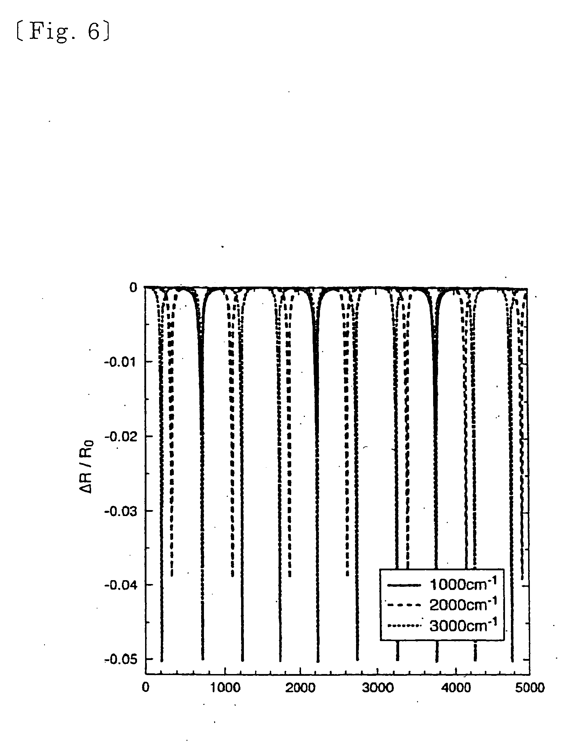

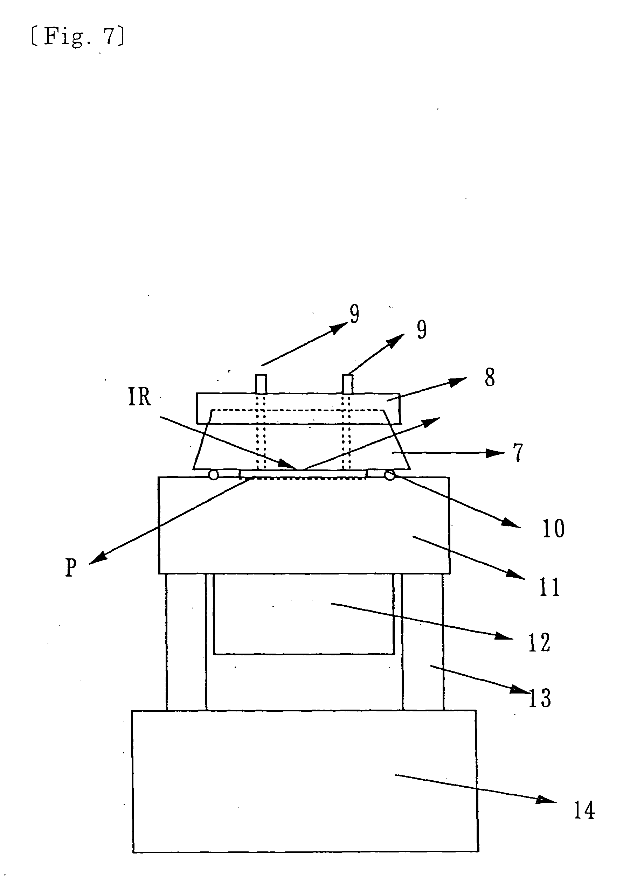

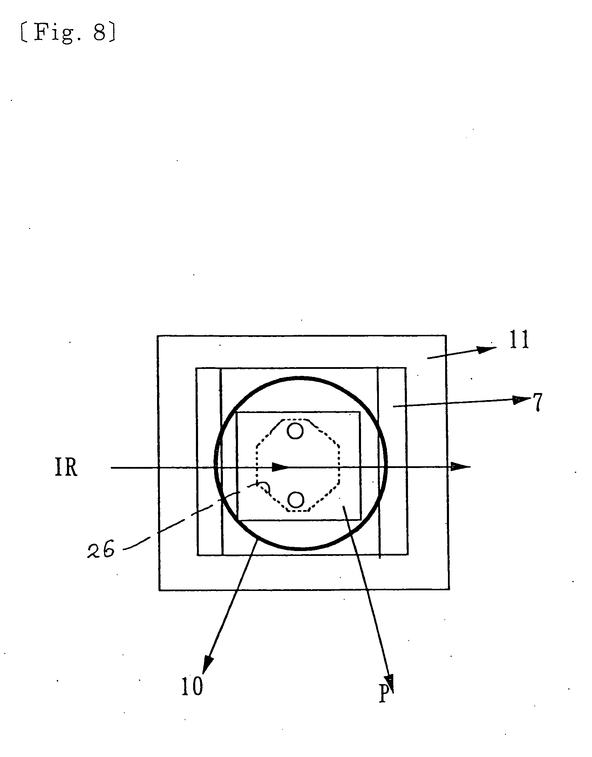

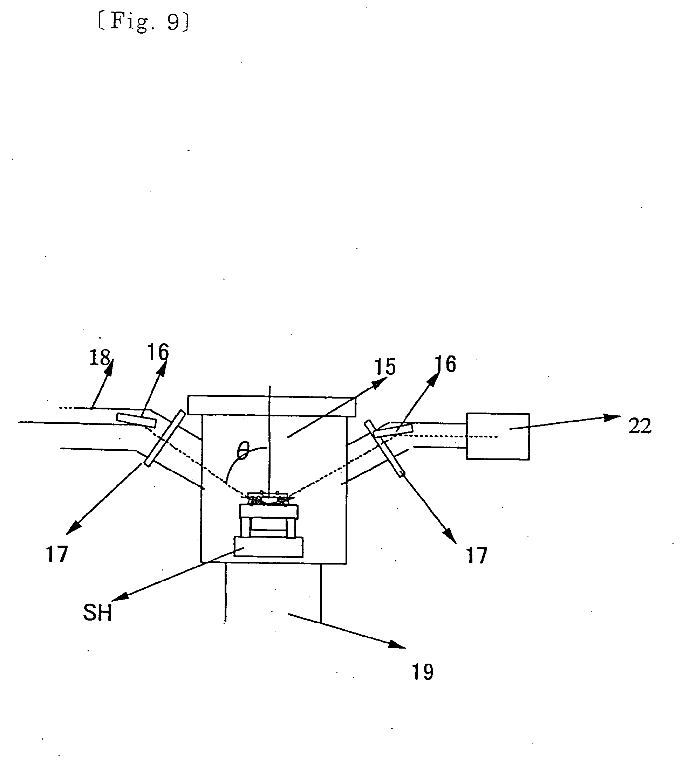

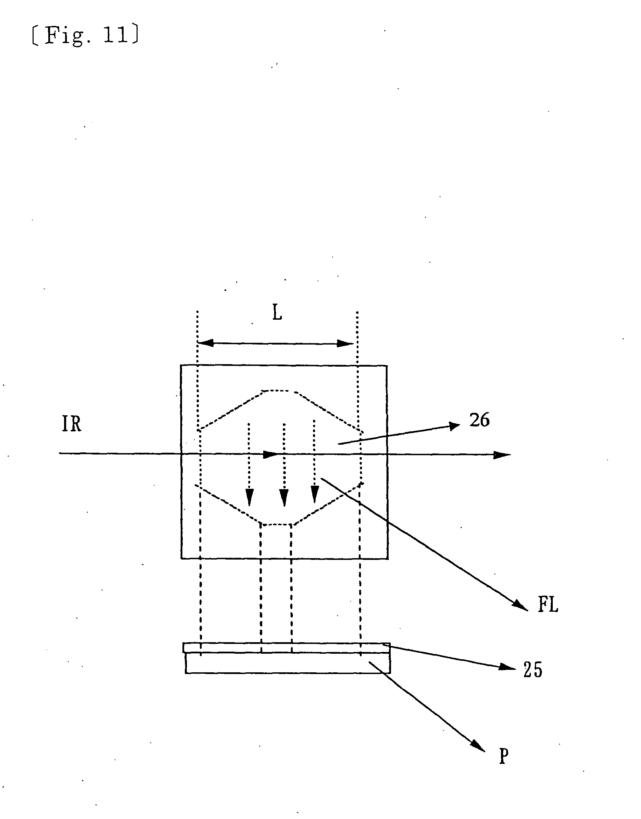

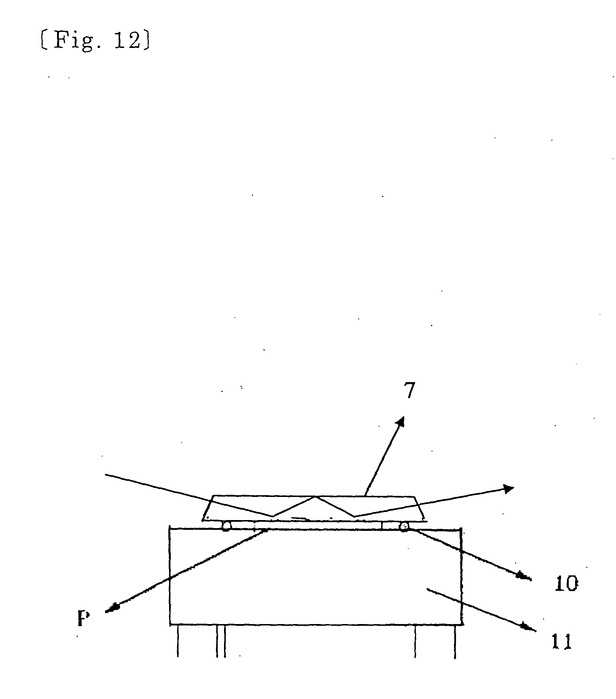

Fig. 1 is a diagram showing one embodiment of protein chip as one example of biochip. Fig. 2 is a diagram showing a degree of irregularity on an active layer surface of a biochip produced in Example. Fig. 3 is a diagram showing an infrared absorption spectrum of avidin. Fig. 4 is a diagram showing an atomic force microscopic image of a surface of a biochip on which avidin is immobilized. Figs. 5 and 6 are diagrams each showing dependency of sensitivity of infrared reflection absorption spectrum measurement of biochip to an active layer thickness. Fig. 7 is a diagram showing one embodiment of sample holder. Fig. 8 is a plan view of Fig. 7. Fig. 9 is a diagram showing one embodiment of biochip analysis apparatus. Fig.
10 is a plan view of Fig. 9. Fig. 11 is a diagram showing another embodiment of biochip. Fig. 12 is a diagram showing a preferred embodiment of biochip analysis method.

### DETAILED DESCRIPTION OF THE INVENTION

Hereinafter, embodiments and examples of the first to the twentieth aspects of this invention will be described in conjunction with the best embodiment and example.

### [Embodiment 1: Protein Chip]

One example of protein chip according to this invention will be described based on Fig. 1. In this protein chip, a metallic layer 2 is provided on a solid base 1, and a thin film active layer 3 made from a nonmetallic material is provided on the metallic layer 2. Amodification layer 4 described later in this specification is formed on a surface portion of the active layer 3. On the modification layer 4, a probe protein 5 capable of adsorbing a test substance 6 (test protein, for example) is immobilized. The protein chip is usable for infrared reflection absorption analysis due to the above-described constitution, which is the great characteristic of the protein chip.

The form and the size of the solid base are not limited. A material to be used for the solid base may be selected arbitrarily, and preferred examples thereof include Si, SiO₂, quartz, and ceramic such as titania.

A material to be used for the metallic layer is not particularly limited, too, and it is possible to use cobalt, chrome, cobalt silicide, platinum, gold, silver, a combination thereof, an alloy thereof, or the like. A thickness of the metallic layer may be set arbitrarily, and the thickness may be about 0.5 to 1 µm, for example. When providing the metallic layer on the solid base, it is possible to employ a known vapor deposition method, sputtering, chemical vapor deposition (CVD), or the like.

The active layer has a film thickness less than an infrared wavelength, more preferably a film thickness sufficiently less than the infrared wavelength. The suitable film thickness of the active layer may vary depending on the infrared light to be used, and a film thickness of 200 nm or less may be preferred for using p-polarization infrared light, while a film thickness of 600 nm or more may be preferred for using s-polarization infrared light.

Though the type of the nonmetallic material for forming the active layer is not particularly limited, a semiconductor material or an electrically insulating material may preferably be used. Examples of the semiconductor material include silicon, gallium arsenide, germanium, and the like. Examples of the electrical insulating material include various ceramics such as silica and alumina; polymer materials, and the like. In providing the active layer on the metallic layer, it is possible to employ vapor deposition, sputtering, CVD, and the like. The surface of the active layer may preferably be highly flat, and a flatness of the surface in terms of difference in elevation may preferably be 5 nm or less, for example.

The surface of the active layer has the role of immobilizing the probe protein. For the role, it is possible to form the surface portion of the active layer as the modification layer having a reaction group as described above, or it is possible to form an organic self assembled monolayer on the surface of the active layer.

In the case of forming the modification layer, the reaction group such as a carboxyl group or an amino group is added to the surface portion of the active layer by various known chemical modification methods. The various chemical modification methods are known, and specific explanations for the methods are omitted. It is possible to immobilize the probe protein by chemical binding with the reaction group.

It is also possible to immobilize the probe protein by avidin-biotin binding by introducing biotin into the probe protein and temporarily binding avidin to the reaction group in the modification layer. Such method is known in the field of protein chip and the like. In the case of using the method, though the process is complicated, an effect of preventing degeneration of the probe protein is great, and it is possible to control orientation of the immobilizedprobe protein, thereby enhancing an effect of adsorbing the test protein.

Preferred examples of the "self assembled organic monolayer" to be formed on the surface of the active layer include a lipid double membrane. Examples of a method of forming the lipid double membrane include the method disclosed in Literature 5 indicated below.

### [Literature 5]

"Discrete membrane arrays" Y. Cheng, S. D. Ogier, R. J. Bushby, S. D. Evans, Molecular Biotechnology 74 (2000) , 159-174

The process is complicated also when employing the method of Literature 5. However, an effect of preventing degeneration of the probe protein is great, and it is possible to control orientation of the immobilizedprobe protein, thereby enhancing an effect of adsorbing the test protein.

### [Example 1]

Hereinafter, one example of biochip production by a chemical modification method (method for forming a metallic layer and an active layer) particularly contrived by the inventors of this invention will be described.

In this example,
(1) a metallic layer is formed on a solid base,
(2) a nonmetallic material is deposited on the metallic layer by vapor deposition, sputtering, or chemical vapor deposition (CVD), and then an active layer made from a nonmetallic material and having a film thickness less than an infrared wavelength and a flatness in terms of difference of elevation of 5 nm or less is formed by annealing, and
(3) a probe is immobilized on a surface of the active layer.

In immobilizing the probe, it is possible to form a modification layer in which a reaction group such as a carboxyl group, an amino group, and the like is attached to the surface by, for example, reacting a silane coupling agent with the surface of the active layer followed by a hydrolysis reaction or the like.

The surface of the active layer obtained above has the flatness in terms of difference of elevation of 5 nm or less. In the case of depositing an amorphous nonmetallic material on the metallic layer, it is not particularly difficult to achieve the flatness in terms of difference in elevation of 1 nm or less. It is possible to immobilize the probe on the surface of such active layer by an appropriate method.

The following is one specific case of this example. On a surface of a silicon-based solid base, a cobalt layer having a thickness of 20 to 100 nm is deposited by sputtering. On the cobalt layer, an amorphous SiO₂ active layer having a thickness of 100 to 250 nm is deposited by sputtering. Then, annealing is performed under a nitrogen atmosphere at 300°C to 800°C for 30 minutes to one hour. A degree of irregularity of a surface of one example of the thus-obtained active layer is shown in Fig. 2, and a difference of elevation thereof is well less than 1 nm, which is a surprising flatness.

Further, an infrared absorption spectrum of avidin of the biochip produced by Example 1 is shown in Fig. 3. This biochip is obtained by carboxylating the surface of the active layer and then immobilizing avidin as the probe protein by a reaction of an amino group with the carboxyl group. In Fig. 3, the spectrum lines a and b are measurement result according to this example. The spectrum lines c and d are measurement results according to Comparative Example wherein avidin is adsorbed by a simple physical adsorption without performing the chemical modification as in the spectrum lines a and b. The spectrum lines a and c are infrared absorption spectrums to vertical polarization, and the spectrum lines b and d are infrared absorption spectrums to horizontal polarization. In Fig. 3, "Absorbance" in the vertical direction indicates a scale of absorbance which is a unit representing a degree of intensity of light absorption by a substance.

In Fig. 3, no difference is observed in peaks of the spectrum lines c and d, and a prominent difference is observed in peaks of the spectrum lines a and b. This means that the peak of the spectrum is shifted by the polarization, i.e. that orientations of the multiple of immobilized probe proteins are identical.

Further, shown in Fig. 4 is an atomic force microscopic image of the surface of the biochip on which avidin is immobilized. Each of molecules of avidin having the size of several nanometers is picked up as a white dot on the black background. To the inventors' knowledge, this image is the world's first image of one molecule of protein measured on a substrate which is usable for measurement of infrared reflection absorption spectrum.

### [Example 2]

As the carboxylation of the active layer surface described in Example 1, a method formed of a first step and a second step described below is particularly excellent.

First Step: The chip of Example 1 on which the annealing is performed is dipped into 0.5 mM/L of a toluene solution of carbomethoxyethyltrichlorosilane at -8°C for one hour. Then, the chip is washed with toluene, acetone, methanol, and pure water in this order.

Second Step: The chip is dipped into a concentrated hydrochloric acid solution for 3 to 8 hours for hydrolysis.

### [Example 3]

According to the seventh aspect of this invention, sensitivity of the infrared reflection spectrum measurement, i. e. dependency of ΔR/R activity to the active layer thickness, of the biochip on which the SiO₂ active layer is formed as in Example 1 was measured at each of three types of wavelengths 1,000 cm⁻¹, 2,000 cm⁻¹, and 3000 cm⁻¹. The results are shown in Figs. 5 and 6. Shown in Fig. 5 is the case of p-polarization infrared light, and shown in Fig. 6 is the case of s-polarization infrared light. The numbers on the horizontal axis in each of Figs. 5 and 6 are thicknesses of the active layer indicated by the unit of nanometer.

As is apparent from Fig. 5, in the case of the p-polarization, it is possible to perform the measurement with high sensitivity at all the wavelengths insofar as the thickness of the active layer is about 200 nm or less. In turn, as is apparent from Fig. 6, in the case of the s-polarization, the sensitivity is 0 when the thickness of the active layer is low. The sensitivity is increased cyclically along with the increase in thickness, and the peaks of the sensitivity vary depending on the wavelengths. However, in general, it is difficult to control a thickness of several hundreds of nanometers or more with accuracy of 100 nm. Therefore, when the thickness is set to 600 nm or more, the s-polarization absorption appears at any wavelengths due to fluctuation in thickness.

That is, in the case of conventional gold substrates, a thickness of a self assembled monolayer used as the active layer is typically several nanometers to several tens of nanometers, which is relatively thin. Therefore, the conventional gold substrate is sensitive only to the p-polarization and can not be used for the s-polarization measurement. According to this invention, it is easy to form the active layer having the thickness of 600 nm or more, and it is possible to perform the s-polarization measurement. Therefore, it is possible to identify the protein and the like with higher accuracy.

### [Embodiment 2: Protein Chip Analysis Apparatus and Protein Chip Analysis Method]

The protein chip analysis apparatus according to this invention is formed at least of the above-described protein chip, a liquid passing unit for supplying a sample liquid containing a test substance to the protein chip, and an optical system for entering infrared light to the protein chip and detecting reflected infrared light.

It is preferable that the protein chip is disposed in a space capable of realizing a substantially vacuum state. The optical system may preferably include a prism to be disposed on the protein chip, but it is possible to use a reflection mirror or a light guide tube such as a glass fiber in place of the prism. Further, the liquid passing unit may preferably be formed of a liquid reservoir in the protein chip provided with the spacer layer and a liquid passing structure provided with a liquid passage for supplying and discharging the sample liquid to the liquid reservoir. Needlesstosay, itisnecessary to exercise extreme caution for ensuring vacuum seal in a structure of a joint portion of the liquid passing unit.

The protein chip analysis method according to this invention is performed by using the above-described protein chip analysis apparatus and performing at least the following steps.
(1) A step of entering infrared light from the optical system to a protein chip which has not been brought into contact with a test substance and measuring reflected infrared light as a background of an infrared reflection absorption spectrum. In this step, an appropriate liquid which is not the sample liquid is supplied to the liquid reservoir. As such liquid, a buffer solution is particularly preferred in order to prevent degeneration of the probe protein. It is possible to select the buffer solution arbitrarily from various buffer solutions such as HEPES, and it is possible to use a buffer solution of which pH is controlled by adding thereto a CaCl₂, NaCl, NaOH, or the like.
(2) A step of supplying the sample liquid to the protein chip to cause the test substance in the sample liquid to be adsorbed when contacting the probe protein and then entering infrared light to the protein chip to measure reflected infrared light as a signal of an infrared reflection absorption spectrum.
(3) A step of obtaining a spectrum which is a ratio between the signal and the background and then identifying the test substance by using the spectrum as an infrared reflection absorption spectrum of the test substance.

The method of measuring the background and the signal of the infrared reflection absorption spectrums to obtain the spectrum of the ratio therebetween and identifying the test substance from the spectrum is the method usually employed in measurement of infrared spectrum.

It is particularly preferable to perform the entering of the infrared light and the collection of the reflected infrared light in (1) and (2) via a prism. It is particularly preferable to set an incident angle of the infrared light entered to the protein chip to 60° or more of grazing incidence. Further, it is particularly preferable to repeat the step of guiding the infrared light entered to the liquid reservoir and reflected by the biochip further to the liquid reservoir to cause the infrared light to be reflected (again) by the protein chip at least once.

One example of the protein chip analysis apparatus according to this invention will be described based on Figs. 7 and 8. As shown in Fig. 7, a protein chip P is placed on a predetermined position of a sample stage 11, and an O-ring 10 is placed in such a fashion as to enclose the protein chip P. The O-ring 10 is made from a material capable of elastic deformation and has a thickness larger than that of the protein chip P. A prism 7 capable of transmitting infrared light is placed on the protein chip P.

The prism 7 is brought into a close contact with a top face of the protein chip P in a state of compressing the O-ring 10 by using a holding clasp 8. Therefore, it is possible to insulate the protein chip P from the outside in an air tight fashion by the sample stage 11, the prism 7, and the O-ring 10.

On an active layer of the protein chip P, a spacer layer formed by hollowing out a space in the form indicated by the broken line in Fig. 8 is provided. A liquid reservoir 26 is formed on the active layer of the protein chip P by the spacer layer. A liquid passage 9 connected to the liquid reservoir 26 is provided in the prism 7 and the holding clasp 8. The above-described liquid passing unit is formed of the above components, and a sample liquid containing a test substance (or a buffer solution) is supplied to and discharged from the liquid reservoir 26 of the protein chip P using the liquidpassing unit.

By forming the liquid passing unit in this way, the structure of a so-called microchannel in which the protein reaction system is kept in a narrow space is established. Therefore, the advantage of reducing an amount of the sample liquid required for the measurement to a remarkably small amount is achieved, and, more importantly, it is possible to perform a protein molecular recognition reaction and to measure the infrared absorption spectrum in a solution system capable of preventing degeneration of the protein.

The sample stage 11 is supported by a support column 13 on a support board 14. Under the sample stage 11, a peltiert element 12 for controlling a temperature of the sample stage 11 and the protein chip P is attached. The apparatus as a whole shown in Figs. 7 and 8 will hereinafter be referred to as a sample holder SH.

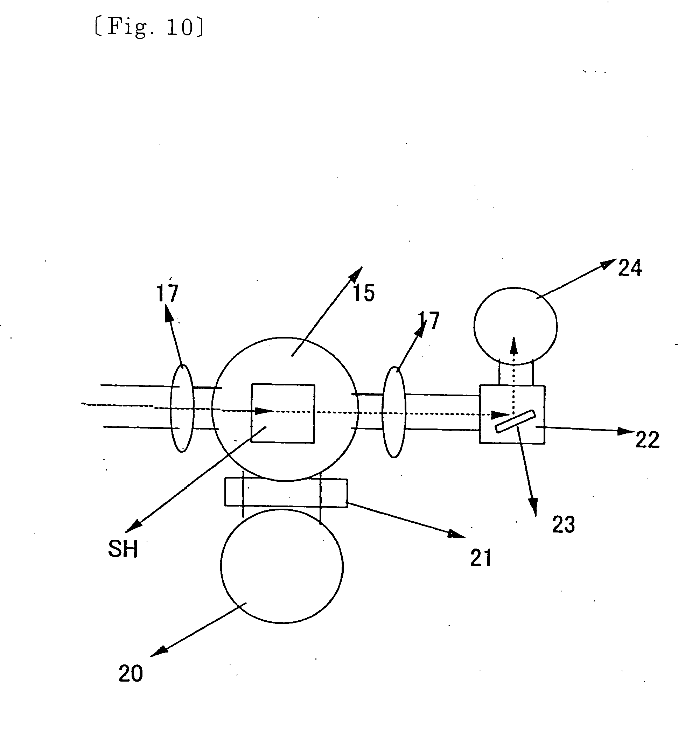

As shown in Figs. 9 and 10, the sample holder SH is disposed inside a main vacuum chamber 15. An infrared light beam (IR) outputted from a Fourier transform infrared spectroscopy device (FTIR) (not shown) is entered to a surface of the protein chip P after passing through a duct 18 and via a light collection mirror 16. Denoted by 19 is an exhaust duct.

An incident angle of the infrared light beam is an important parameter that greatly influences on the sensitivity. For example, a case of using a transparent prism of BaF₂ at a low frequency near 800 cm⁻¹ will be described. At a wavelength 6 µm at a water absorption peak position, refraction factors of water and BaF₂ are 1.25 and 1. 45, respectively. Accordingly, when the incident angle on the bottom of the prism is set to 55°, 57°, or 58°, the incident angle of the infrared light beam on the surface of the protein chip is 71.9°, 76.6°, or 79.6°.

In the infrared reflection absorption spectroscopy using the metallic layer, the incident angle of 80° to 85° is desirable. In view of the above, it is desirable to set the incident angle (θ of Fig. 4) of the infrared light beam on the prism bottom face to about 58°. When the incident angle is excessively large, the infrared light is reflected by the prism bottom face, so that the infrared light reaching the protein chip can be weakened to deteriorate the sensitivity. In the case where θ is 58°, for example, the reflection loss is about 19% (calculated value) at a wavelength of 6 µm.

Since CaF₂ has stronger resistance to water as compared to BaF₂, CaF₂ can be used as the prism material when the lower limit of the measurement region of near 1,000 cm-1 is not disadvantageous. The incident angle can be set by the calculation same as that when using BaF₂. For example, the incident angle of the infrared light beam on the prism bottom face is 71° since the refraction factor of CaF₂ is a little less than that of BaF₂. In this case, an incident angle under water is about 83°.

A focus distance of the light collection mirror 16 is so set that the infrared light is collected on the surface of the protein chip P. A window plate 17 of BaF₂ may be attached for the purpose of insulating the vacuum state of the main vacuum chamber 15 from the duct 18 when so required.

The infrared light reflected on the protein chip P is collected in a detector 24 by a light collection mirror 23 disposed in a mirror chamber 22. As the detector 24, a mercury cadmium telluride detector and the like may be used.

As shown in Fig. 10 which is a plan view of Fig. 9, a mechanism wherein an air lock chamber 20 is connected to the main vacuum chamber 15 via an air tight valve 21 so that the sample holder SH reciprocates between the main vacuum chamber 15 and the air lock chamber 20 may be used. For example, in the case of assembling the sample holder SH or exchanging the protein chip P, the following procedure is performed. The sample holder SH is moved to the air lock chamber 20 followed by closing the air tight valve 21, and then a predetermined work is performed by releasing the vacuum state of the air lock chamber 20. Then, the air lock chamber 20 is tightly sealed followed by opening the air tight valve 21, and deaeration is performed after returning the sample holder SH to the main vacuum chamber 15. By performing the above procedure, it is possible to largely reduce the time required for achieving a necessary vacuum degree of the main vacuum chamber 15.

### [Embodiment 3: Modification Example of Protein Chip]

In Fig. 11, a plan view of a protein chip is shown in the upper part, and a front view of the protein chip is shown in the lower part. In the protein chip shown in Fig. 11, a spacer layer 25 obtained by hollowing out a space in the form indicated by the broken line is provided on a top face (i.e., active layer) of the protein chip P having the structure shown in Fig. 1. A liquid reservoir 26 is formed on the active layer of the protein chip P by the spacer 25.

How to use method and advantages in use of the protein chip shown in Fig. 11 have been described in conjunction with Figs. 7 and 8, and the thickness of the spacer layer 25 (consequently, thickness of the liquid reservoir 26) is the important parameter in the above usage from the reasons described in conjunction with the ninth aspect of this invention.

Under assumption that the thickness of the spacer layer 25 is 1 µm and a width of a liquid flow (width L in Fig. 11) in the liquid reservoir 26 is 10 mm, infrared light absorption by water in the liquid reservoir 26 was evaluated. At a wavelength of 6 µm of a water absorption peak position, an absorption factor α is 0.2742 × 10⁴. When the incident angle of the infrared light beam on the prism surface is 55°, 57°, or 58°, the incident angle of the infrared light beam on the protein chip surface is 71.9°, 76.6°, or 79.6°. Accordingly, loss by the water absorption is 83%, 91%, or 95%.

It is important to perform coating with Teflon (trade name) to which proteins hardly attach in the case of forming the liquid passing unit shown in Figs. 7 and 8, or, more specifically, it is important to perform the coating with Teflon on the prism bottom face, the liquid passage, and the like with which the sample liquid is brought into contact. Also, from the viewpoint that the protein chip has the microchannel structure, it is easy to construct a structure which is provided also with other functions such as synthesis and analysis, without limitation to the simple liquid passage structure as shown in Fig. 7 and 8.

### [Embodiment 4: Modification Example of Use of Protein Chip]

In the embodiment shown in Fig. 12, the infrared light irradiated onto and then reflected by the liquid reservoir of the protein chip P returns to the prism 7 to be totally reflected by the prism 7, and the protein chip P is irradiated with the totally reflected infrared light, so that the infrared absorption spectrum of protein is measured for the plural times. With this method, it is possible to enhance measurement sensitivity.

## Claims

1. A biochip production method comprising the steps of:
(1) forming a metallic layer on a solid base;
(2) depositing a non-metallic material on the metallic layer by vapor deposition, sputtering, or chemical vapor deposition (CVD) and then forming, by performing annealing, an active layer made from a non-metallic material and having a film thickness lower than an infrared wavelength and a surface flatness in terms of difference of elevation of 5 nm or less; and
(3) immobilizing a probe on the surface of the active layer.

2. The biochip production method according to claim 1, wherein the annealing is performed under an inert gas atmosphere at a temperature of 300°C to 800°C.

3. The biochip production method according to claim 1 or claim 2, wherein a semiconductor material or an electrical insulating material is used as the nonmetallic material.

4. A biochip comprising:
a solid base; a metallic layer provided on the solid base; an active layer provided on the metallic layer and made from a nonmetallic material; and a probe immobilized on a surface of the active layer, wherein
the active layer has a film thickness less than an infrared wavelength and a surface flatness in terms of difference of elevation of 5 nm.

5. The biochip according to claim 4, wherein the probe is a protein, a polynucleotide, a sugar chain, or a cell.

6. The biochip according to claim 4 or claim 5, wherein the active layer is made from a semiconductor material or an electrical insulating material.

7. The biochip according to any one of claim 4 to claim 6, wherein the active layer has a film thickness of 200 nm or less in the case of using p-polarization infrared light and has a film thickness of 600 nm or more in the case of using s-polarization infrared light.

8. The biochip according to any one of claim 4 to claim 7, wherein a modification layer having a reaction group is formed on a surface portion of the active layer, and the probe is immobilized by a chemical binding with the reaction group.

9. The biochip according to any one of claim 4 to claim8, wherein the probe is a protein; a lipid double membrane is formed on the surface of the active layer; and the probe is immobilized in a state where the probe is embedded in the lipid double membrane.

10. The biochip according to any one of claim 4 to claim 9, wherein a spacer layer formed by hollowing out a space for liquid reservoir formation is provided on the active layer.

11. The biochip according to claim 10, wherein the spacer layer has a uniform thickness in the range of 0.2 to 100 µm.

12. A biochip analysis apparatus comprising:
the biochip defined in any one of claim 4 to claim 11; a liquid passing unit for supplying a sample liquid containing a test substance to the biochip; and an optical system entering infrared light to the biochip and detecting reflected infrared light.

13. The biochip analysis apparatus according to claim 12, wherein the optical system further comprises a prism provided on the biochip.

14. The biochip analysis apparatus according to claim 12 of claim 13, wherein the biochip is disposed in a space capable of realizing a substantially vacuum state.

15. The biochip analysis apparatus according to any one of claim 12 to claim 14, wherein the liquid passing unit is formed of the liquid reservoir in the biochip provided with the spacer layer and a liquid passing structure provided with a liquid passage for supplying and discharging the sample liquid to and from the liquid reservoir.

16. A biochip analysis method using the biochip analysis apparatus defined in any one of claim 12 to claim 15, comprising the steps of:
(1) measuring, as a background of an infrared reflection absorption spectrum, reflected infrared light of infrared light entered from the optical system of the biochip analysis apparatus to a biochip which has not contacted a test substance;
(2) supplying a sample liquid to the biochip to cause the test substance in the sample liquid to be adsorbed when contacting a probe and then measuring reflected infrared light of infrared light entering the biochip as a signal of an infrared reflection absorption spectrum; and
(3) obtaining a spectrum which is a ratio between the signal and-the background and then identifying the test substance by using the spectrum as an infrared reflection absorption spectrum of the test substance.

17. The biochip analysis method according to claim 16, wherein the entering of the infrared light and the collection of the reflected infrared light of (1) and (2) are performed via the prism.

18. The biochip analysis method according to claim 16 or claim 17, wherein an incident angle in entering the infrared light to the biochip is set to 60° or more of grazing incidence.

19. The biochip analysis method according to any one of claim 16 to claim 18, wherein the sample liquid of (2) is supplied to the liquid reservoir in the biochip provided with the spacer layer, and the process of guiding the infrared light entered to the liquid reservoir and reflected by the biochip further to the liquid reservoir so as to cause the infrared light to be reflected again by the biochip is repeated at least once.

20. The biochip analysis method according to claim 19, wherein the unit for guiding the reflected infrared light further to the liquid reservoir is the prism provided on the biochip.
